# EUROPEAN PATENT APPLICATION

(11) **EP 4 336 518 A1**
(43) Date of publication of application: **13.03.2024**
(21) Application number: 22315232.3
(22) Date of filing: 07.09.2022
(51) Int. Cl.: G16H 50/30

(54) **DATA PROCESSING SYSTEM AND METHOD FOR PREDICTING A SCORE REPRESENTATIVE OF A PROBABILITY OF A SEPSIS FOR A PATIENT**

(71) Applicant: Previa Medical, 69002 Lyon (FR)
(72) Inventor: Thiboud, Pierre-Elliott, 69100 Villeurbanne (FR); Arribe, Barthélémy, 69009 Lyon (FR); Francois, Quentin, 69002 Lyon (FR)
(74) Representative: Bringer IP

(57) **Abstract**

The invention relates to data processing system for predicting a score representative of a probability of a sepsis for a patient, comprising a data interface (12) configured to receive, from at least one database (114, 116), health data of at least one patient, characterized in that the data processing system comprises a trained machine learning model (24) configured to predict and provide the score using as input the health data for each patient, and to provide a plurality of sub-scores representative of a correlation between the health data and the predicted score, the health data comprising regularly updated biometric monitoring data provided by a biometric monitoring device and health history data provided by at least one health history database.

## Description

### 1. Technical field of the invention

The invention relates to a system and method for monitoring hospitalized patients and for predicting a score representative of a probability of an occurrence of a sepsis. The invention also relates to the training of a machine learning model for said monitoring and prediction.

### 2. Technological background

Sepsis is a medical term describing a general inflammatory response associated to a severe infection. The infection cause may be bacterial, fungal, parasitic, or viral and can be linked to approximatively 20% of global death worldwide.

The medical response to a sepsis is time sensitive as it is estimated that each hour of delay in antimicrobial administration is associated to an average decrease in survival of 7,6% (see Kumar, A., et al., Duration of hypotension before initiation of effective antimicrobial therapy is the critical determinant of survival in human septic shock. Critical care medicine, 2006. 34(6): p. 1589-1596).

Sepsis prevention and response has been recognized as global health priority by the World Health Organization in 2017. One of the objectives is to diagnose sepsis as early as possible to administrate the antimicrobial as soon as possible, thus raising the recovery rate of the patient. The optimal response would be to diagnose the occurrence of a sepsis even before any visible or measurable symptoms.

One way to diagnose a sepsis is the calculation of a score called SOFA score for Sequential Organ Failure Assessment score. The SOFA score is used to track a patient status by monitoring several health parameters which are used to calculate the score.

As the SOFA score increase, the rate of organ failure rises and a medical intervention is needed to prevent complication or death of the patient.

As the SOFA score is used to determine the organ failure which can be an indicator of the presence of a sepsis, method have been sought to predict the score in order to anticipate a future health deterioration and administrate a medical treatment as soon as possible, even in absence of visible symptoms.

Some solutions to predict a score representative of a probability of an organ failure for a patient to predict sepsis or other life-threatening condition have been proposed. However, such solution often failed to provide the medical personnel with enough indication on why such a score is predicted, as they are designed as opaque system unable to explain the score to the medical personnel. If the score predicts the occurrence of a sepsis in a near future without giving enough explanation, the medical personnel could consider that the score has been miscalculated and not take any actions to treat the case, fearing a false alert.

Moreover, most of the score prediction is based only on health monitoring data and do not make differences between patients with different preexisting conditions and having an history of a sepsis during a previous hospitalization.

The inventors have therefore sought a solution which makes it possible to overcome at least some of the aforementioned constraints.

### 3. Aims of the invention

The invention aims to provide a data processing system for predicting a score representative of a probability of a sepsis: an organ failure for a patient caused by an infection.

The invention aims in particular to provide a data processing system able to provide an explanation of the score provided to the medical personnel.

The invention also aims to provide a data processing system able to take as input previous hospitalization of the patient.

The invention also aims to provide a data processing system able to take as input preexisting condition of a patient.

### 4. Disclosure of the invention

To do this, the invention relates to data processing system for predicting a score representative of a probability of a sepsis for a patient, comprising a data interface configured to receive, from at least one database, health data of at least one patient, characterized in that the data processing system comprises a trained machine learning model configured to predict and provide the score using as input the health data for each patient, and to provide a plurality of sub-scores representative of a correlation between the health data and the predicted score, the health data comprising regularly updated biometric monitoring data and health history data provided by at least one health history database.

A data processing system of the invention allows a monitoring of each patient which takes into account not only the biometric monitoring data but also the health history data from the patient, based on a trained machine learning which use the same monitoring and health history data. This allows the system to evaluate the score and determine a probability of occurrence of a sepsis long before any apparition of symptoms and with a better performance than the existing system. The machine learning model, or machine learning algorithm, can be any machine learning type suitable for the application, and can be for example a gradient boosting algorithm, or a neural network, etc.

Moreover, a plurality of sub-score is calculated to give more depth to the calculated score such that the medical personnel could control the pertinence of the predicted score and thus improve the confidence in the relevance of the score and the confidence in the data processing system which calculated the score.

The monitoring data are preferably provided by a biometric monitoring device and/or documented manually by a person from the medical personnel.

The biometric monitoring device can for example be one or more of a patch, a watch, a bracelet, a belt, glasses, a ring, an implant, etc. Each biometric monitoring device can provide with one or several biometric monitoring data, and each patient can be monitored by a person from the medical personnel and/or by one or several biometric monitoring devices.

According to a particularly advantageous variant of the invention, the health history data can also comprise previous laboratory results included in an history of laboratory results.

According to a particularly advantageous variant of the invention, the health data can also comprise every clinical information available related to the patient.

According to a particularly advantageous variant of the invention, the data processing system comprises a module for receiving health history data from at least one health history database, each history database being an internal health history database providing health history data from a hospital or an external health history database providing health history data centralized from multiple sources of health history data.

In particular, the health history data can be provided by an internal health history database stored by the hospital which operates the data processing system and/or be provided by an external health history database centralizing data related to the patient from multiple sources and stored in an external server managed by a centralized entity, for example a regional, provincial, federal, national, or international centralized health database storing health data of the patient.

According to a particularly advantageous variant of the invention, one or more sub-scores are comprised in the following list of sub-scores:
- temperature,
- heart rate,
- oxygen saturation,
- diastolic pressure,
- systolic pressure,
- respiratory rate,
- health history,
- age of the patient,
- lactate level,
- leukocyte level,
- platelet level,
- bilirubin level,
- urine output during the last 24h,
- creatinine level,
- partial pressure of oxygen in arterial blood (Pₐ0₂),
- fraction of inspired oxygen (F_{I}O₂),
- Glasgow Coma Score,
- perioperative complications,
- surgery procedure,
- effective operation duration,
- planned operation duration,
- type of surgery.

The invention therefore makes it possible to provide one, several or all these sub-scores to the medical personnel which can get a better overview of the calculated score. For example, a patient hospitalized may have biometric monitoring data that may appear relatively good, in comparison to other patients. But the score may be high due to health history data, if the patient had an occurrence of sepsis in a previous hospitalization while having the same evolution of biometric monitoring data, or if the patient has a preexisting disease. The medical personnel can thus see a better explanation of the score by checking the sub-scores which can be displayed on a screen.

Depending on the sub-score and the health history data, the word "level" can signify for example a concentration or a count of a component measured by laboratory analysis, for example present in a fluid of the patient (for example blood or urine).

Any laboratory results which can be analyzed for the patient can be used as a sub-score for the system when it is used as an input for the prediction of the score.

According to a particularly advantageous variant of the invention, each or every clinical information available which is used as input parameter can be used as a sub-score.

According to a particularly advantageous variant of the invention, sub-scores can be grouped into a new sub-score to simplify understanding of the invention by the medical personnel and reduce the amount of information to process.

According to a particularly advantageous variant of the invention, the data processing system comprises a module for calculating the sub-scores, configured to compute for each input of the machine learning model the positive or negative weight of said input on the predicted score, and to provide a list of most relevant sub-scores.

The list of most relevant is for example classified from most positive weight to most negative weight. The list of most relevant sub-scores can also be classified by absolute value of the weights.

The invention therefore makes it possible to provide a local explainability of the machine learning model by analyzing and understanding the weight of each input for the calculation of the predicted score, for example by using a value explainability model.

Alternatively, or in a complementary way, a global explanation can be computed by the module for calculating the sub-scores, the data processing system thus being able to provide the most relevant sub-scores to predict if the patient has a sepsis.

The explainability, local or global, can be intrinsic as it is easily understandable from the machine learning model. The explainability can also be a retroactive analysis in which the internal functioning is estimated after the prediction of the score, i.e. an a posteriori analysis.

According to a particularly advantageous variant of the invention, the data processing system comprises a display device configured to display at least the predicted score and at least one sub-score.

The invention therefore makes it possible to provide the medical personnel with the score and at least one sub-score, preferably in a way easily interpretable for the medical personnel.

According to a particularly advantageous variant of the invention, the display device is' configured to display at least the predicted score and a predetermined number of first sub-scores on the list of most relevant sub-scores.

The invention therefore makes it possible for the medical personnel to see the most relevant sub-scores and such have a vision of the explainability of the predicted score. The predetermined number of first sub-scores taken from the list is for example comprised between five and ten sub-scores. This allows to display only the sub-scores which have the greatest relevance on the predicted score. The relevance, also called impact, depends on the ranking criterium for the list, for example the most relevant sub-score can be those with positive weights with the greatest values or those with positive and negative weights with the greatest absolute values.

Advantageously and according to the invention, the system comprises an alert device configured to send an alert if the score of a patient is over a predetermined threshold.

The invention therefore makes it possible to alert the medical personnel if the score is too high, indicating a high probability of a sepsis.

Advantageously and according to the invention, the data interface is configured to transform the health data to a HL7^{®} FHIR^{®} resource.

The invention therefore makes it possible to be interfaced with any equipped hospital by obtaining the data directly formatted in an interoperable format.

The invention also relates to a method of training a machine learning model of a data processing system according to the invention, characterized in that the input training data comprise health data representative from at least one previous hospitalization history from a plurality of patients, and for each patient:
- at least one history of biometric monitoring data over each period of hospitalization,
- data representative of occurrence or absence of a sepsis and the severity of any occurrence of a sepsis by the patient during the period of hospitalization.

The training method use data from at least one previous hospitalization history as a way to improve the prediction of the score when the data processing system is used on a patient.

Advantageously and according to the invention, the input training data comprise, for each patient, health history data from the patient.

The invention therefore makes it possible to produce a better prediction compared to the method of a prior art which focused on biometric monitoring data.

Advantageously and according to the invention, the input training data are regularly updated and the method of training comprises a step of updating the machine learning model by training the machine learning model with the updated training data.

The step of updating the machine learning model may be carried out for example at each reception of updated input data, or when a predetermined size of updated input data is received, or at a predetermined time interval, etc.

This update of the machine learning model allows the machine learning model to improve with new input data, thus improving the predicted score.

The invention also relates to a data processing system and a training method characterized in combination by some or all of the characteristics mentioned above or below.

### 5. List of figures

Further aims, features and advantages of the invention can be found in the following description, which is provided solely as a non-limiting example, and which refers to the accompanying figures, in which:
- Figure 1 is a schematic view of a data processing system from an embodiment of the invention,
- Figure 2 is a schematic view of a display of a data processing system from an embodiment of the invention,
- Figure 3 is a schematic view of a training method from an embodiment of the invention.

### 6. Detailed description of one embodiment of the invention

For the sake of illustration and clarity, the drawings do not strictly adhere to scales and proportions.

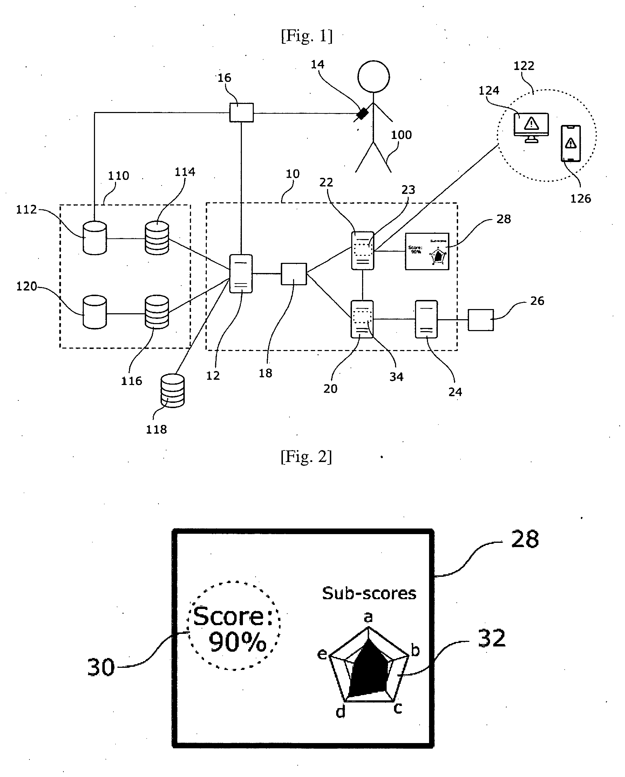

Figure 1 is a schematic view of a data processing system 10 from an embodiment of the invention, for predicting a score representative of a probability of a sepsis for a patient 100.

The data processing system 10 comprises a data interface 12 configured to receive, from at least one database, health data of at least one patient. The health data comprise regularly updated biometric monitoring data provided by a biometric monitoring device 14, especially a biometric monitoring device worn by the patient. The biometric monitoring device can for example be a patch, a watch, a bracelet (as represented in figure 1), a belt, glasses, a ring, an implant, etc. The biometric data can be processed by a pre-processing module 16 and can provide the data directly to the data interface 12 or to a data system 112 from the hospital infrastructure 110 in which the patient 100 is hospitalized. The data system 112 can for example be an electronic health record (EHR) system which aggregate the data during the hospitalization and store them in a first hospital database 114.

The health data comprise also health history data provided by a at least one health history database 116, 118. In this embodiment, the health history data are provided by a second hospital database 116 which is connected to an internal health history data system 120 from the hospital infrastructure or an external centralized data system 118, aggregating history data from several sources (not shown) such as practician database, pharmacy database, laboratory database, etc.

For example, in France, the internal health history data system 120 is called a PMSI system (for *Programme de medicalisation des systemes d'information* in French, program for medicalization of information system) and the centralized database is the *Espace Numérique de Santé* (health digital space).

In order to ensure interoperability of the health data coming from the plurality of databases, the data interface 12 is configured to transform the health data to a HL7^{®} FHIR^{®} resource (FHIR^{®} being a registered trademark and an acronym for Fast Healthcare Interoperability Resources).

The data received by the data interface 12 are treated by a security module 18 and provided as data inputs to two modules, a prediction module 20 and a frontend module 22.

The prediction module 20 is a backend module, here integrated in a backend server, and is configured to predict the score and provide a plurality of sub-scores representative of a correlation between the health data and the predicted score based on the provided data inputs. The prediction of the score and the computation of sub-scores is based on a machine learning model 24 trained with input training data 26 comprising health data representative from at least one previous hospitalization history from a plurality of patients, and for each patient:
- at least one history of biometric monitoring data over each period of hospitalization, and health history data from the patient.
- data representative of occurrence or absence of a sepsis and the severity of any occurrence of a sepsis by the patient during the period of hospitalization.

The input data can be provided by the same type of sources as the data used by the data processing system 10 to predict the score, i.e. health data from a plurality of patients from one or several hospitals, and from a centralized database 118. Input training data can be regularly updated and the machine learning model is updated by training the machine learning model with the updated training data.

The frontend module 22, here integrated in a frontend server, is configured to provide the predicted score and computed sub-scores to a display screen 28 and comprises an alert module 23 to provide alerts to medical personnel if the predicted score is above a predetermined threshold, for example alerts appearing on the display screen 28 or alerts sent to personal devices 122 of medical personnel such as personal computers or laptops 124, phones or smartphones 126, pagers, etc.

Figure 2 is a schematic view of a display screen 28 of a data processing system from an embodiment of the invention.

The display screen 28 can be used to enable medical personnel to see at least the predicted score 30 here expressed as a percentage, and some computed sub-scores 32, especially the sub-scores which are the most relevant. The display screen 38 can also display further information (not shown for clarity purpose), for example information commonly displayed to the medical staff, especially administrative information to identify the patient, live biometric data, time, and date, etc.

The most relevant sub-scores 32 can be selected from a list of most relevant sub-scores classified from most positive weight to most negative weight for the prediction of the score, as described further below. The most relevant sub-scores are presented to the medical personnel in a way that can be easily interpreted, for example as represented in figure 1 and 2, a radar chart presenting the five more relevant sub-scores named here a, b, c, d, e for illustration, and the weight of each sub-score for the prediction of the displayed score. The radar chart can be adapted to display more sub-scores.

The sub-scores can for example be based on data inputs for the machine learning model or combination of data inputs grouped together. A non-exhaustive list of sub-score can be: temperature, heart rate, oxygen saturation, diastolic pressure, systolic pressure, respiratory rate, laboratory results, health history, age of the patient, lactate concentration, leukocyte concentration, platelet count, bilirubin concentration, diuresis, creatinine level, partial pressure of oxygen in arterial blood (Pa02), fraction of inspired oxygen (FIO2), Glasgow Coma Score, number of intraoperative complications, operation denomination, actual operation duration, expected operation duration, type of surgery, etc.

The computation of the positive or negative weight of the inputs uses a SHAP value explainability model (for SHapley Additive explanation), a method based on the Shapley value and for example available for the gradient boosting algorithm which can be used to implement the machine learning algorithm.

Back to figure 1, the data processing system 10 includes a module 34 for calculating the sub-scores, preferably integrated in the prediction module 22, configured to compute for each input of the machine learning model the positive or negative weight of said input on the predicted score, and to provide the list of most relevant sub-scores classified from most positive weight to most negative weight.

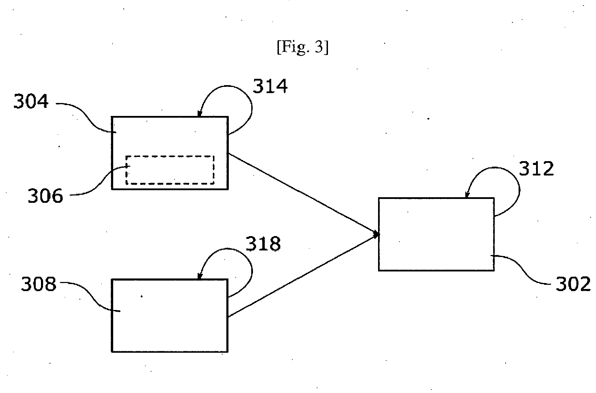

Figure 3 is a schematic view of a training method from an embodiment of the invention. The training method 300 comprises a step 302 of training the machine learning model of the data processing system, characterized in that the input training data comprise health data representative from at least one previous hospitalization history from a plurality of patients, and for each patient:
- at least one history 304 of biometric monitoring data over each period of hospitalization,
- data 306 representative of occurrence or absence of a sepsis and the severity of any occurrence of a sepsis by the patient during the period of hospitalization.

The input training data comprise also, for each patient, health history data 308 from the patient.

The step 302 of training can be executed periodically and/or regularly which enables an update 312 of the machine learning model. The update 312 of the machine learning model can be triggered by the reception of an update 314 of the history of biometric monitoring data and/or by the reception of an update 318 of the health history data.

## Claims

1. A data processing system for predicting a score (30) representative of a probability of a sepsis for a patient, comprising a data interface (12) configured to receive, from at least one database (114, 116), health data of at least one patient, **characterized in that** the data processing system comprises a trained machine learning model (24) configured to predict and provide the score (30) using as input the health data for each patient, and to provide a plurality of sub-scores representative of a correlation between the health data and the predicted score (30), the health data comprising regularly updated biometric monitoring data and health history data provided by at least one health history database.

2. A data processing system according to claim 1, **characterized in that** it comprises a module for receiving health history data from at least one health history database (116, 118), each history database being an internal health history database (116) providing health history data from a hospital or an external health history database (118) providing health history data centralized from multiple sources of health history data.

3. A data processing system according to any of the claims 1 or 2, **characterized in that** one or more sub-scores are comprised in the following list of sub-scores:
- temperature,
- heart rate,
- oxygen saturation,
- diastolic pressure,
- systolic pressure,
- respiratory rate,
- health history,
- age of the patient,
- lactate level,
- leukocyte level,
- platelet level,
- bilirubin level,
- urine output during the last 24h,
- creatinine level,
- partial pressure of oxygen in arterial blood (Pa02),
- fraction of inspired oxygen (FIO2),
- Glasgow Coma Score,
- perioperative complications,
- surgery procedure,
- effective operation duration,
- planned operation duration,
- type of surgery.

4. A data processing system according to any of the claims 1 to 3, **characterized in that** it comprises a module (34) for calculating the sub-scores, configured to compute for each input of the machine learning model (24) the positive or negative weight of said input on the predicted score, and to provide a list of most relevant sub-scores.

5. A data processing system according to any of the claims 1 to 4, **characterized in that** it comprises a display device (28) configured to display at least the predicted score (30) and at least one sub-score.

6. A data processing system according to a combination of claim 4 and 5, **characterized in that** the display device (28) is configured to display at least the predicted score (30) and a predetermined number of first sub-scores (32) on the list of most relevant sub-scores.

7. A data processing system according to any of the claims 1 to 6, **characterized in that** it comprises an alert module (23) configured to send an alert if the score of a patient is over a predetermined threshold.

8. A data processing system according to any of the claims 1 to 7, **characterized in that** the data interface (12) is configured to transform the health data to a HL7^{®} FHIR^{®} resource.

9. Method of training a machine learning model (24) of a data processing system according to claims 1 to 8, **characterized in that** the input training data comprise health data representative from at least one previous hospitalization history from a plurality of patients, and for each patient:
- at least one history of biometric monitoring data over each period of hospitalization,
- data representative of occurrence or absence of a sepsis and the severity of any occurrence of a sepsis by the patient during the period of hospitalization.

10. Method of training a machine learning model (24) according to claim 9, **characterized in that** the input training data comprise, for each patient, history data from the patient.

11. Method of training a machine learning model (24) according to any of claims 9 to 10, **characterized in that** the input training data are regularly updated and **in that** it comprises a step of updating the machine learning model (24) by training the machine learning model with the updated training data (26).
